Europäisches Patentamt

European Patent Office

Office européen des brevets

(19)

(11) Publication number: 0 322 215 B2

# NEW EUROPEAN PATENT SPECIFICATION

(12)

(45) Date of publication of the new patent specification: 30.11.94

(51) Int. Cl.5: C07C 53/08, C07C 51/42

(21) Application number: 88312118.8

(22) Date of filing: 21.12.88

(54) **Purification of acetic acid with ozone.**

(30) Priority: 23.12.87 US 137844

(43) Date of publication of application:
28.06.89 Bulletin 89/26

(45) Publication of the grant of the patent:
26.08.92 Bulletin 92/35

(45) Mention of the opposition decision:
30.11.94 Bulletin 94/48

(84) Designated Contracting States:
BE DE ES FR GB IT NL SE

(56) References cited:
EP-A- 0 161 874
DE-A- 1 568 833
DE-A- 2 415 700
GB-A- 1 423 485
JP-A- 5 564 545

PATENT ABSTRACTS OF JAPAN, unexamin-
ed applications, C field, vol. 10, no. 219, July
31, 1986; THE PATENT OFFICE JAPANESE
GOVERNMENT, page 40 C 363

CHEMICAL ABSTRACTS, vol. 85, no. 20, No-
vember 15, 1976, Columbus, Ohio, USA; R.H.
WALTER et al.: "Duration of ozone in water
in the upper solubility range" page 454, col-
umn 1, abstract no.149 605p

(73) Proprietor: HOECHST CELANESE CORPORA-
TION
Route 202-206 North
Somerville, N.J. 08876 (US)

(72) Inventor: Scates, Mark Owen
1801 Crooked Creek
Pearland Texas (US)
Inventor: Gibbs, Russell K., Jr.
2007 Firtree Way
Houston Texas (US)
Inventor: Torrence, G. Paull
301 South Morningside
Corpus Christi Texas (US)

(74) Representative: De Minvielle-Devaux, Ian
Benedict Peter et al
CARPMAELS & RANSFORD
43, Bloomsbury Square
London WC1A 2RA (GB)

EP 0 322 215 B2

Rank Xerox (UK) Business Services
(3.10/3.09/3.3.3)

**Description**

Field of the Invention

This invention relates to the purification of acetic acid and relates more particularly to the purification of acetic acid resulting from the catalytic carbonylation of methanol.

DESCRIPTION OF THE PRIOR ART

Various methods have been employed for producing acetic acid including, for example, the oxidation of acetaldehyde, the oxidation of petroleum naphtha, butane or the like, or the direct synthesis from methanol and carbon monoxide. One of the more useful commercial methods for the production of acetic acid is the carbonylation of methanol as disclosed in U.S. 3,769,329. The carbonylation catalyst comprises rhodium, either dissolved or otherwise dispersed in a liquid reaction medium or else supported on an inert solid, along with a halogen-containing catalyst promoter as exemplified by methyl iodide. The rhodium can be introduced into the reaction system in any of many forms, and it is not relevant, if indeed it is possible, to identify the exact nature of the rhodium moiety within the active catalyst complex. Likewise, the nature of the halide promoter is not critical. A large number of suitable promoters are disclosed, most of which are organic iodides. Typically, the reaction is conducted with the catalyst being dissolved in a liquid reaction medium through which carbon monoxide gas is continuously bubbled.

An improvement in the prior-art process for the carbonylation of an alochol to produce the carboxylic acid having one carbon atom more than the alcohol in the presence of a rhodium catalyst is disclosed in copending, commonly assigned application U.S. Serial No. 699,525, filed February 8, 1985 and European patent application 161,874; published November 21, 1985. As disclosed therein, acetic acid (HAc) is produced from methanol (MeOH) in a reaction medium comprising methyl acetate (MeOAc), methyl halide, especially methyl iodide, (MeI), and rhodium present in a catalytically-effective concentration. The invention therein resides primarily in the discovery that catalyst stability and the productivity of the carbonylation reactor can be maintained at surprisingly high levels, even at very low water concentrations, i.e. 4 wt.% or less, in the reaction medium (despite the general industrial practice of maintaining approximately 14 wt.% or 15 wt.% water) by maintaining in the reaction medium, along with a catalytically-effective amount of rhodium, at least a finite concentration of water, methyl acetate and methyl iodide, a specified concentration of iodide ions over and above the iodide content which is present as methyl iodide or other organic iodide. The iodide ion is present as a simple salt, with lithium iodide being preferred. The applications teach that the concentration of methyl acetate and iodide salts are significant parameters in affecting the rate of carbonylation of methanol to produce acetic acid especially at low reactor water concentrations. By using relatively high concentrations of the methyl acetate and iodide salt, one obtains a surprising degree of catalyst stability and reactor productivity even when the liquid reaction medium contains water in concentrations as low as about 0.1 wt.%, so low that it can broadly be defined simply as "a finite concentration" of water. Furthermore, the reaction medium employed improves the stability of the rhodium catalyst, i.e. resistance to catalyst precipitation, especially during the product-recovery steps of the process wherein distillation for the purpose of recovering the acetic acid product tends to remove from the catalyst the carbon monoxide which in the environment maintained in the reaction vessel, is a ligand with stabilizing effect on the rhodium. U.S. Serial No. 699,525 is herein incorporated by reference.

The acetic acid which is formed by the carbonylation of methanol is converted to a high purity product by conventional means such as by a series of distillations. While it is possible in this manner to obtain acetic acid of relatively high purity, the acetic acid product contains a considerable amount of by-product impurities, determinable on the basis of their reducing action on permanganate. The amount of such reducing impurities is referred to as the permanganate time. Since the permanganate time is an important commercial test which the acid product must meet for many uses, the presence therein of such impurities is highly objectionable. Apparently the removal of minute quantities of these impurities by conventional rectification alone is difficult since the impurities distil over with the acetic acid.

Among the residual impurities which have been found to degrade the permanganate time are alkyl iodide impurities which are most likely carried over into the product stream from the catalyst solution in the reactor. Also found in the acetic acid product are various unsaturated and carbonyl impurities including crotonaldehyde, ethyl crotonaldehyde and the 2-methyl-2-pentenal isomer thereof. As has been previously stated, it is both difficult and costly to remove the iodides, unsaturates and carbonyl impurities from the acetic acid product by physical methods since such impurities are present in such minute amounts. Accordingly, an economical process for removing such impurities is needed.

2

Various methods have been suggested to purify or remove nonacidic components from carboxylic acids. For example, U.S. 4,576,683 discloses a method of separating $C_1$-$C_{10}$ aliphatic and $C_3$-$C_{10}$ olefinic carboxylic acids from mixtures with nonacids by extractive distillation using an amide as an extractant to recover an extractant-acid mixture followed by recovery of the acids from the extractant-acid mixture by rectification. The method disclosed in the patent is described as being particularly suitably applied on aqueous mixtures of formic, acetic, and/or propionic acid which mixtures contain unconverted hydrocarbons and other oxygenated compounds such as mixtures with alcohols, aldehydes, and/or ketones and which may also contain further contaminants such as effluents from the oxidation reactions. The amide extractants utilized in the patent are selected from lactams having 5 or 6 membered rings. Pyrrolidone and derivatives thereof are specifically disclosed.

U.S. 4,268,362 is concerned with providing a method of removing formaldehyde from raw acetic acid which has been formed by synthetic reactions such as oxidation of acetaldehyde, gas phase or liquid phase oxidation of butane, oxidation of petroleum naphtha or paraffins, as well as the reaction of methanol with carbon monoxide. The separation process involves treating the acetic acid in a heating zone at a temperature at about the boiling point of the acetic acid or higher, removing the heated product and delivering it to a distillation zone and operating the distillation zone so as to obtain a lower boiling fraction, a higher boiling fraction and an intermediate acetic acid fraction which will have a formaldehyde content of 300 ppm or lower.

U.S. 3,725,208 is concerned with a process for the removal of small amounts of aldehyde impurities from acrylic acids which comprises adding to the acrylic acid minor amounts of a compound selected from the group consisting of sulfuric acid, hydrazine, phenyl hydrazine, aniline, monoethanolamine, ethylene diamine and glycine and subjecting the acrylic acid mixture to distillation. Although hydrazine usually reacts exothermically with acrylic acid to form pyrrazolidone, and amines such as monoethanolamine and ethylene diamine have the properties of forming salts and aminocarboxylic acids with acrylic acid, the patentee states that it was surprising that these compounds react predominantly with aldehydes contained in acrylic acid and can remove them from the acrylic acid.

Japanese patent application 84-176729, assigned to Daicel Chemical Industries, Ltd., discloses purification of acetic acid by adding a small amount of peracetic acid to raw acetic acid, heating the mixture at 50°C. to 120°C. for about 20 hours, and thereafter subjecting the mixture to distillation.

Japanese patent application 60-222439 discloses purification of acetic anhydride produced by the ketene process in which acetic acid is thermally cracked to ketene which then combines with acetic acid through an absorption reaction to produce acetic anhydride.

An additional patent teaching improving the permanganate time of aliphatic acids is U.S. Patent 2,900,413 which discloses a heating and distillation step.

## SUMMARY OF THE INVENTION

The present invention is a method for purifying acetic acid which has been produced by the low water carbonylation of methanol in a reaction medium containing 0.5-30 wt % methyl acetate, 5-20 wt % methyl iodide, and 2-20 wt % alkali metal iodide in the presence of a halogen-promoted rhodium catalyst and in the presence of less than 14 wt % water, the thus-produced acetic acid containing halide, unsaturate and carbonyl impurities, by which method the "permanganate time" is improved, characterised in that the said acid is contacted with ozone of sufficient quantity to oxidise the said impurities and thereafter recovering the purified acetic acid.

Thus acetic acid formed and recovered from the catalytic carbonylation of methanol, can be purified of minute amounts of unsaturates, iodides, and carbonyl compounds by treatment with ozone which reacts with such impurities. The ozone-derived impurities may be subsequently separated from the acetic acid, if desired, by conventional distillation or adsorption on activated carbon, molecular sieves or an ion-exchange resin.

## DETAILED DESCRIPTION OF THE INVENTION

The ozonolysis treatment of the present invention is applicable to the purification of acetic acid which has been produced by the carbonylation of methanol in the presence of a metal catalyst such as rhodium. The purification process of the present invention is particularly useful when the carbonylation reaction is catalyzed by rhodium and a halide promoter such as an organic halide disclosed in U.S. 3,769,329. The process of purifying acetic acid in the present invention is more particularly useful when the acetic acid is formed by the carbonylation of methanol under low water conditions such as set out in U.S. Serial No.

699,525 wherein the catalyst solution not only contains the rhodium catalyst and organic halide promoter, but also contains an additional iodide salt. It has been found that organic iodide impurities as well as unsaturated and carbonyl impurities degrade the commercial value of the acetic acid product.

In the low water carbonylation of methanol to acetic acid as exemplified in U.S. Serial No. 699,525, the catalyst which is employed includes a rhodium component and a halogen promoter in which the halogen is either bromine or iodine. Generally, the rhodium component of the catalyst system is believed to be present in the form of a coordination compound of rhodium with a halogen component providing at least one of the ligands of such coordination compound. In addition to the coordination of rhodium and halogen, it is also believed that carbon monoxide ligands form coordination compounds or complexes with rhodium. The rhodium component of the catalyst system may be provided by introducing into the reaction zone rhodium in the form of rhodium metal, rhodium salts and oxides, organic rhodium compounds, coordination compounds of rhodium, and the like.

The halogen promoting component of the catalyst system consists of a halogen compound which is methyl iodide.

The liquid reaction medium employed may include any solvent compatible with the catalyst system and may include pure methanol, or mixtures of the methanol feedstock and/or the desired acetic acid and/or esters of these two compounds. The preferred solvent and liquid reaction medium for the low water carbonylation process comprises the acetic acid product. Thus, the preferred solvent is acetic acid.

Water is also added to the reaction medium, but, at concentrations well below what has heretofore been thought practical for achieving sufficient reaction rates. It is known that in rhodium-catalyzed carbonylation reactions of the type set forth in this invention, the addition of water exerts a beneficial effect upon the reaction rate (U.S. Patent No. 3,769,329). Thus, commercial operations run at water concentrations of at least 14 wt.%. Accordingly, it is quite unexpected that reaction rates substantially equal to and above reaction rates obtained with such high levels of water concentration can be achieved with water concentrations below 14 wt.% and as low as 0.1 wt.%.

It has been found that under low water concentrations, methyl acetate and lithium iodide act as rate promoters only when relatively high concentrations of each of these components are present and that the promotion is higher when both of these components are present simultaneously. This has not been recognized in the prior art previous to disclosure of commonly assigned U.S. Serial No. 699,525. The concentration of lithium iodide used in the reaction medium of the preferred carbonylation reaction system is believed to be quite high as compared with what little prior art there is dealing with the use of halide salts in reaction systems of this sort.

The carbonylation reaction may be carried out by intimately contacting the feed methanol, which is in the liquid phase, with gaseous carbon monoxide bubbled through a liquid reaction medium containing the rhodium catalyst, halogen-containing promoting component, methyl ester, and additional soluble iodide salt promoter, at conditions of temperature and pressure suitable to form the carbonylation product. The feed is methanol, the halogen-containing promoting component comprises methyl iodide and the methyl ester comprises methyl acetate. The iodide salt is an iodide salt of a member of the group consisting of the metals of Group Ia of the periodic table as set forth in the "Handbook of Chemistry and Physics" published by CRC Press, Cleveland, Ohio, 1975-76 (56th edition). In particular, alkali metal iodides are useful, with lithium iodide being preferred. In the low water carbonylation useful in this invention, the additional iodide over and above the organic iodide promoter is present in the catalyst solution in amounts of from 2-20, preferably 10-20 wt.%, the methyl acetate is present in amounts of from 0.5 -30, preferably 2-5 wt.%, and the methyl iodide is present in amounts of from 5-20, and 14-16 wt.%. The rhodium catalyst is present in amounts of from 200-1000 and preferably 300-600 ppm.

Typical reaction temperatures for carbonylation will be approximately 150-250°C, with the temperature range of about 180-220°C being the preferred range. The carbon monoxide partial pressure in the reactor can vary widely but is typically about 2-30 atmospheres, and preferably, about 4-15 atmospheres. Because of the partial pressure of by-products and the vapor pressure of the contained liquids, the total reactor pressure will range from about 15 to 40 atmospheres.

A reaction and acetic acid recovery system which can be employed, within which the present improvement is used, comprises (a) a liquid-phase carbonylation reactor, (b) a so-called "flasher", and (c) a "methyl iodide-acetic acid splitter column". The carbonylation reactor is typically a stirred autoclave within which the reacting liquid contents are maintained automatically at a constant level. Into this reactor there are continuously introduced fresh methanol, sufficient water to maintain at least a finite concentration of water in the reaction medium, recycled catalyst solution from the flasher base, and recycled methyl iodide and methyl acetate from the overhead of the methyl iodide-acetic acid splitter column. Alternate distillation systems can be employed so long as they provide means for recovering the crude acetic acid and recycling to the reactor catalyst solution, methyl iodide, and methyl acetate. In the preferred process, carbon monoxide is continuously introduced into the carbonylation reactor just below the agitator which is used to stir the contents. The gaseous feed is, of course, thoroughly dispersed through the reacting liquid by this means. A gaseous purge stream is vented from the reactor to prevent buildup of gaseous by-products and to maintain a set carbon monoxide partial pressure at a given total reactor pressure. The temperature of the reactor is controlled automatically, and the carbon monoxide feed is introduced at a rate sufficient to maintain the desired total reactor pressure.

Liquid product is drawn off from the carbonylation reactor at a rate sufficient to maintain a constant level therein and is introduced to the flasher at a point intermediate between the top and bottom thereof. In the flasher the catalyst solution is withdrawn as a base stream (predominantly acetic acid containing the rhodium and the iodide salt along with lesser quantities of methyl acetate, methyl iodide, and water), while the overhead of the flasher comprises largely the product acetic acid along with methyl iodide, methyl acetate, and water. A portion of the carbon monoxide along with gaseous by-products such as methane, hydrogen, and carbon dioxide exits the top of the flasher.

The product acetic acid drawn from the base of the methyl iodide-acetic acid splitter column (it can also be withdrawn as a side stream near the base) is then drawn off for final purification such as to remove water as desired by methods which are obvious to those skilled in the art including, most preferably, distillation. The overhead from the methyl iodide-acetic acid splitter, comprising mainly methyl iodide and methyl acetate, is recycled to the carbonylation reactor along with fresh methyl iodide, the fresh methyl iodide being introduced at a rate sufficient to maintain in the carbonylation reactor the desired concentration of methyl iodide in the liquid reaction medium. The fresh methyl iodide is needed to compensate for losses of methyl iodide in the flasher and carbonylation reactor vent streams.

The crude dry acetic acid product is not adequately purified since it contains residual by-products such as organic and metal iodides, unsaturates, and carbonyl impurities of which crotonaldehyde, ethyl crotonaldehyde, and 2-methyl-2-pentenal are the most prominent. Small amounts of these impurities degrade the commercial usefulness of the acetic acid product and accordingly it has been discovered that by treating the acetic acid with ozone it becomes possible to obtain a desired degree of purification as evidenced by the permanganate test.

According to one aspect of the invention, the crude acetic acid is subject to ozonolysis by generating the ozone gas and bringing the gas into physical contact with the acetic acid product. In another aspect of the invention, ozonolysis of the acetic acid is carried out in the presence of a catalytically effective amount of an oxidation catalyst.

Ozone ($O_3$) is a gaseous allotropic form of oxygen in which three atoms form the molecule rather than the normal two. Although ozone is a strong oxidizing agent, it is not a specific oxidant and hence will oxidize any material it contacts which has a lower oxidation potential. As such, when it contacts the aforesaid impurities in acetic acid, it will oxidize the carbon to carbon double bond linkages of unsaturates, for example, which apparently contribute to short permanganate times in the acetic acid product. This theory of operation, however, is not to be regarded as essential to an understanding of the invention. Available data, as shown hereinafter, indicate that the benefit of the ozone contacting is due to the ability of the ozone to render iodides, unsaturates, and carbonyl compounds inactive, thus preventing their influence on acetic acid either during storage or subsequent use.

Ozonolysis may be carried out by generating the ozone from any suitable source such as a quartz lamp, a silent electric discharge or spark discharge commonly called corona discharge, but it is preferable to obtain the ozone from a source of radiation in the range between about 1000 and 2950 angstrom units in wave length, applied in air or oxygen. For commercial production of ozone, it is preferable to use corona discharge technology on either air or oxygen. UV radiation type generators are usually only used on a small scale system. The maximum weight ratio of ozone in the liquid acetic acid is governed by the flammability limits of acetic acid - $O_2$ vapor phase compositions. In the examples hereinafter set forth, ozone was introduced into the mid point of a cylindrical vessel and contacted with a downwardly flowing stream of acetic acid at a temperature of about 75°F. (24°C.) Sufficient pressure was employed to keep the acetic acid below the flammability limit of 2.5 volume per cent in oxygen or 3.8 volume per cent in air (~8 psig. in air or 12 psig in $O_2$).

The ozone exposure time will vary, but it has been found that the effect is substantially instantaneous, while on the other hand, over exposure is not harmful. Good results are obtained when the exposure time is less than one-half hour, usually about 1 to 15 minutes. The preferred quantity of ozone will range from about 3 ppm to 5000 ppm based on the weight of the acetic acid treated. High levels of ozone are not detrimental except for associated costs.

The ozonolysis treatment may be carried out catalytically in the presence of about 0.001 to 5.0 wt % of any known oxidation catalyst which does not dissolve in acetic acid. Suitable catalysts are metal or metal oxides of elements selected from the group of manganese, platinum, palladium, silver, rhodium, ruthenium, rare earth (cerium), nickel, chromium, cobalt, and the like. If desired, the catalyst may be mixed with a suitable carrier such as diatomaceous earth, activated carbon, silica, silicon carbide, alumina, zeolites (faujasite) or the like. The catalyst is preferably used in a fixed bed reactor and the ozonolysis may be carried out at temperatures of 70°F. (21°C.) to 125°F. (52°C.) in a continuous or batchwise fashion. Temperature and pressure considerations are not critical so long as flammability limits are not exceeded.

The iodides, unsaturates and carbonyl impurities in acetic acid apparently react with ozone to form a reactive oxygenated species or complex which may, if desired, be separated from the acetic acid. Such separation can be accomplished by passing the solution through activated carbon, molecular sieve zeolites, including shape selective zeolites, or an ion exchange resin in H + form or by distilling the pure acetic acid from the complex. Upon carbon treatment of the acetic acid product obtained from the carbonylation of methanol with ozone, the permanganate time of the acetic acid is greatly improved compared to ozone treatment alone. Removal of iodide compounds from acetic acid by means of macroreticulated strong-acid cation exchange resins is described in U.S. 4,615,806, hereby incorporated by reference.

The present invention can be more fully understood by referring to the following examples which illustrate the best mode now contemplated for carrying out the invention. In the examples the "permanganate time" is determined as follows:

One ml of an aqueous 0.1 N potassium permanganate solution is added to 50 ml of acetic acid in a graduated cylinder at room temperature. The cylinder is stoppered and shaken, and a timer is immediately started to measure the time required for the purple color to change to a yellow-amber end point which is compared to a standard reference color indicating the content of unsaturate, iodide and carbonyl impurities.

## EXAMPLE 1

185 grams of glacial finished acetic acid (obtained from a low water carbonylation of methanol to acetic acid employing a halogen promoted rhodium catalyst) spiked to contain 231 ppm crotonaldehyde and 224 ppm ethyl crotonaldehyde impurities and having a permanganate time of 0.1 minutes were treated for 30 minutes with ozone made from air (0.5 vol.% ozone in air).

## EXAMPLE 2

Unspiked glacial finished acetic acid was treated with ozone in the manner of Example 1 in which the contact time with ozone was 10 and 19 minutes.

## EXAMPLE 3

Glacial finished acetic acid was spiked with ethyl crotonaldehyde (23.2 ppm) and treated with ozone in the manner of Example 1.

## EXAMPLE 4

A glacial acetic acid overhead cut identified as T-840H was treated with ozone and compared to an untreated sample.

## EXAMPLE 5

Two samples of glacial acetic acid, identified as V-783 outlet, were treated with ozone at different contact times of 1 and 3 minutes and compared to an untreated sample.

## EXAMPLE 6

A sample of the untreated glacial acetic acid used in Example 5 was treated with ozone in the presence of 0.5 wt.% manganese dioxide catalyst in two different runs and compared to a sample that was treated with ozone in the absence of a catalyst and a sample that was treated with 0.5% manganese dioxide and air.

The test results of Examples 1 to 6 and the parameters of the tests are set forth below in Table I.

## TABLE I

| Ex. | HAC | Crot. (ppm) | Et Crot. (ppm) | KMnO₄ Time (min.) | Peroxide Concen. (ppm) | O₃ Contact Time (min.) | Feed O₃ Concen. Based. on HAC (ppm) | O₃ Concen. in air (%) | O₃ Gener. Rate (mg/min.) | Total Gas Cont. (l) | Total O₃ Cont. (mg) | Wt. of HAC Treated (gm) |
|---|---|---|---|---|---|---|---|---|---|---|---|---|
| 1 | Spiked HAC blank* | 231 | 224 | 0.1 | -- | -- | -- | -- | -- | -- | -- | 185.0 |
|  | Spiked HAC + O₃* | 9 | 3 | 0.25 | -- | 30 | -- | -- | -- | -- | -- | 185.0 |
| 2 | HAC blank | 5.2 | 5.0 | 1.5 | -- | -- | 0 | -- | -- | 0 | 0 | 183.2 |
|  | HAC + O₃ (10 min.) | 0.1 | 0.6 | -- | -- | 10 | 42 | 0.09 | 0.77 | 4.0 | 7.7 | 183.2 |
|  | HAC + O₃ (19 min.) | 0.1 | 0.5 | 5.0 | < 3 | 19 | 88 | 0.09 | 0.82 | 8.1 | 15.6 | 177.9 |
| 3 | Spiked HAC** | 6.2 | 23.2 | 0.25 | -- | -- | 0 | -- | -- | 0 | 0 | 198.9 |
|  | "  " 2 min O₃ | 0.1 | 0.1 | -- | -- | 2 | 56 | 0.6 | 5.5 | 0.86 | 11.1 | 198.9 |
|  | "  " 4 min O₃ | 0.1 | 0.5 | -- | -- | 4 | 114 | 0.6 | 5.5 | 1.72 | 22.1 | 193.7 |
|  | "  " 5 min O₃ | 0.1 | 0.3 | 1.0 | < 3 | 5 | 146 | 0.6 | 5.5 | 2.15 | 27.6 | 188.4 |
| 4 | T-8404H blank | 6.4 | 5.2 | 1.0 | -- | -- | -- | -- | -- | -- | -- | -- |
|  | T-8404H + O₃ | 0.1 | 0.1 | 8 | -- | 1.0 | 22 | 0.60 | 4.1 | 0.32 | 4.1 | 190.0 |
| 5 | V-783 blank | 6.2 | 7.1 | 0.75 | -- | -- | -- | -- | -- | -- | -- | -- |
|  | V-783+O₃ | .1 | .1 | 3 | -- | 1.0 | 34 | 0.4 | 6.2 | 0.73 | 6.2 | 182.22 |
|  | V-783+O₃ | .1 | .1 | 3 | -- | 3.0 | 26 | 0.1 | 1.6 | 2.05 | 4.9 | 191.22 |
| 6 | V-783+O₃ + MnO₂ | .1 | .1 | 4 | -- | 1.0 | 6 | .07 | 1.1 | 0.74 | 1.1 | 188.91 |
|  | V-783+O₃ | .1 | .1 | 3.25 | -- | 6 | 36 | .05 | 1.1 | 2.5 | 6.8 | 190.21 |
|  | V-783+O₃ + MnO₂ | .1 | .1 | 12 | -- | 6 | 36 | .05 | 1.1 | 2.5 | 6.8 | 187.60 |
|  | V-783+air + MnO₂ | 6.0 | 7.3 | 0.75 | -- | -- | -- | 0 | 0 | 2.5 | 0 | 185.0 |

* Spiked with Crotonaldehyde and Ethyl Crotonaldehyde
** Spiked with Ethyl Crotonaldehyde.

EXAMPLES 7 to 13

In the following examples, improvement in permanganate time is further achieved by ozonolysis followed by activated carbon treatment. A one-half gallon sample of ozone treated acetic acid (obtained from low water carbonylation of methanol to acetic acid employing a halogen promoted rhodium catalyst) was passed through activated carbon in a flooded or trickle bed system at ambient room temperature and pressure. The results are shown in Table 2 below.

TABLE 2

| Example | Permanganate Time, Min. |
|---|---|
| 7. untreated glacial acetic acid | 0.25 |
| 8. ozone treated acid | 1.5 |
| 9. ozone treated acid & activated carbon* (Flooded Bed) | 2.5 |
| 10. ozone treated acid & activated carbon* (Trickle Bed) | 4.5 |
| 11. ozone treated acid & activated carbon* (Flooded contactor) | 2.0 |
| 12. ozone treated acid & activated carbon** (Trickle Bed) | > 10.0 |
| 13. untreated glacial acetic acid & activated carbon** | 0.25 |

\* Calgon F300
\*\* Cocoanut Charcoal

## Claims

1. A method for purifying acetic acid which has been produced by the low water carbonylation of methanol in a reaction medium containing 0.5-30 wt % methyl acetate, 5-20 wt % methyl iodide, and 2-20 wt % alkali metal iodide in the presence of a halogen-promoted rhodium catalyst and in the presence of less than 14 wt % water, the thus-produced acetic acid containing halide, unsaturate and carbonyl impurities, by which method the "permanganate time" is improved, characterised in that the said acid is contacted with ozone of sufficient quantity to oxidise the said impurities and thereafter recovering the purified acetic acid.

2. The method of claim 1 wherein the quantity of ozone contacted is greater than 3 ppm based on the weight of acetic acid treated.

3. The method of claim 1 or 2 wherein the acetic acid is contacted with ozone in the presence of an oxidation catalyst.

4. The method of claim 3 wherein the amount of catalyst is within the range of 0.0001 to 5.0 weight per cent based on the acetic acid.

5. The method of claim 3 or 4 wherein the catalyst is an oxide of rhodium.

6. The method of any of claims 1-5 wherein the purified acetic acid product is recovered by a process involving contact with activated carbon to separate the oxidized halide, unsaturate and carbonyl impurities.

7. A method for preparing acetic acid by the rhodium-catalyzed carbonylation of methanol in the presence of water and a halide promoter as defined in claim 1 characterised in that the acetic acid thus produced is purified by the method of any of claims 1-6.

## Patentansprüche

1. Verfahren zur Reinigung von Essigsäure, die durch Niedrig-Wasser-Carbonylierung von Methanol in einem Reaktionsmedium, das 0,5 bis 30 Gew.-% Methylacetat, 5 bis 20 Gew.-% Methyliodid und 2 bis 20 Gew.-% Alkalimetalliodid enthält, in Gegenwart eines durch Halogen geförderten Rhodium-Katalysators und in Gegenwart von weniger als 14 Gew.-% Wasser hergestellt ist, wobei die so produzierte Essigsäure Halogenid-, ungesättigte und Carbonyl-Verunreinigungen enthält, wobei durch das Verfahren die "Permanganat-Zeit" verbessert wird, dadurch gekennzeichnet, daß die Säure mit Ozon in einer für die Oxidation der genannten Verunreinigungen ausreichenden Menge in Berührung gebracht wird und danach die gereinigte Essigsäure isoliert wird.

2. Verfahren nach Anspruch 1, worin die Menge des Ozons, mit der in Berührung gebracht wird, größer als 3 ppm, bezogen auf das Gewicht der behandelten Essigsäure, ist.

3. Verfahren nach Anspruch 1 oder 2, worin die Essigsäure mit Ozon in Gegenwart eines Oxidations-Katalysators in Berührung gebracht wird.

4. Verfahren nach Anspruch 3, worin die Menge des Katalysators im Bereich von 0,0001 bis 5,0 Gew.-%, bezogen auf die Essigsäure, liegt.

5. Verfahren nach Anspruch 3 oder 4, worin der Katalysator ein Oxid des Rhodiums ist.

6. Verfahren nach irgendeinem der Ansprüche 1 bis 5, worin das gereinigte Essigsäure-Produkt durch ein Verfahren isoliert wird, das den Kontakt mit Aktivkohle einschließt, um die oxidierten Halogenid-, ungesättigten und Carbonyl-Verunreinigungen abzutrennen.

7. Verfahren zur Herstellung von Essigsäure mittels der durch Rhodium katalysierten Carbonylierung von Methanol in Gegenwart von Wasser und einem Halogenid-Promotor, wie er in Anspruch 1 definiert ist, dadurch gekennzeichnet, daß die auf diese Weise hergestellte Essigsäure mittels des Verfahrens nach irgendeinem der Ansprüche 1 bis 6 gereinigt wird.

**Revendications**

1. Méthode de purification d'acide acétique que l'on a produit par la carbonylation à faible teneur en eau du méthanol dans un milieu réactionnel contenant 0,5-30% en poids d'acétate de méthyle, 5-20% en poids d'iodure de méthyle et 2-20% en poids d'iodure d'un métal alcalin en présence d'un catalyseur de rhodium promu par un halogène et en présence de moins de 14% en poids d'eau, l'acide acétique ainsi produit contenant des impuretés d'halogénures, de produits insaturés et de carbonyles, méthodes par laquelle le "temps de permanganate" est amélioré, caractérisée en ce que ledit acide est mis en contact avec de l'ozone en une quantité suffisante pour oxyder lesdites impuretés et ensuite on récupère l'acide acétique purifié.

2. Méthode de la revendication 1, où la quantité d'ozone contacté est plus importante que 3 ppm en se basant sur le poids de l'acide acétique traité.

3. Méthode de la revendication 1 ou 2, où l'acide acétique est mis en contact avec l'ozone en présence d'un catalyseur d'oxydation.

4. Méthode de la revendication 3, où la quantité du catalyseur est comprise entre 0,0001 et 5,00 pour cent en poids en se basant sur l'acide acétique.

5. Méthode de la revendication 3 ou 4, où le catalyseur est un oxyde de rhodium.

6. Méthode selon l'une quelconque des revendications 1-5, où l'acide acétique purifié produit est récupéré par un procédé impliquant un contact avec du charbon activé pour séparer les impuretés oxydées d'halogénure, produit insaturé et carbonyle.

7. Méthode de préparation d'acide acétique par la carbonylation catalysée au rhodium du méthanol en présence d'eau et d'un halogénure promoteur, caractérisée en ce que l'acide acétique ainsi produit est purifié par la méthode selon l'une quelconque des revendications 1-6.